# EUROPÄISCHE PATENTSCHRIFT

(11) **EP 3 801 277 B1**
(45) Veröffentlichungstag und Bekanntmachung des Hinweises auf die Patenterteilung: **06.07.2022**
(21) Anmeldenummer: 19730289.6
(22) Anmeldetag: 06.06.2019
(51) Int. Cl.: A61B 8/06, A61B 5/00, A61B 5/145, A61B 5/026, A61B 8/08, A61B 8/12, A61M 60/135, A61M 60/205, A61M 60/50

(54) **VORRICHTUNG UND VERFAHREN ZUM BESTIMMEN EINES HERZZEITVOLUMENS FÜR EIN HERZUNTERSTÜTZUNGSSYSTEM**
DEVICE AND METHOD FOR DETERMINATION OF A CARDIAC OUTPUT FOR A CARDIAC ASSISTANCE SYSTEM
DISPOSITIF ET PROCÉDÉ DE DÉTERMINATION D'UN DÉBIT CARDIAQUE POUR UN SYSTÈME D'ASSISTANCE CARDIAQUE

(30) Priorität: 06.06.2018 DE 102018208931
(43) Veröffentlichungstag der Anmeldung: 14.04.2021
(73) Patentinhaber: Kardion GmbH, 70376 Stuttgart (DE)
(72) Erfinder: SCHELLENBERG, Inga, 70191 Stuttgart (DE); SCHLEBUSCH, Thomas, Alexander, 71272 Renningen (DE); SCHMID, Tobias, 70197 Stuttgart (DE)
(74) Vertreter: Pfiz, Thomas
(86) Internationale Anmeldenummer: PCT/EP2019/064783
(87) Internationale Veröffentlichungsnummer: WO 2019/234152

(56) Entgegenhaltungen:
- DE-A1-102011 106 142
- US-A1- 2008 097 595
- US-A1- 2016 045 165

## Beschreibung

Die Erfindung geht von einer Vorrichtung oder einem Verfahren nach Gattung der unabhängigen Ansprüche aus.

Um das Herzzeitvolumen eines Patienten mit einem implantierten Herzunterstützungssystem, beispielsweise einem linksventrikulärem Herzunterstützungssystem, zu bestimmen, ist es möglich, einen Pumpenvolumenstrom zu messen, durch eine Korrelation von Betriebsparametern des Herzunterstützungssystems. Die US 2005/0215843 A1 beschreibt ein solches Verfahren. Zusätzlich können zudem physiologische Parameter wie der Blutdruck berücksichtigt werden, wie in der US 2014/0100414 A1 beschrieben. Bei beiden Ansätzen wird der Pumpenvolumenstrom, also ein durch das Herzunterstützungssystem erzeugter Blutfluss gemessen. Dokumente DE102011106142 und US2008097595 beschreiben auch ein implantierbares Herzunterstützungssystem mit einer Sensoreinrichtung.

Ausgehend hiervon liegt der Erfindung die Aufgabe zugrunde, eine Vorrichtung und ein Verfahren zum Bestimmen eines Herzzeitvolumens für ein Herzunterstützungssystem insbesondere auch hinsichtlich der Anordnung und des Einsatzes von Sensoren zu optimieren.

Vor diesem Hintergrund werden mit dem hier vorgestellten Ansatz eine Vorrichtung zum Bestimmen eines Herzzeitvolumens für ein Herzunterstützungssystem, ein Herzunterstützungssystem und ein Verfahren zum Bestimmen eines Herzzeitvolumens gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Mit dem hier vorgestellten Ansatz kann das Herzzeitvolumen eines Patienten mit einem implantierten Herzunterstützungssystem, beispielsweise einem linksventrikulären Herzunterstützungssystem, bestimmt werden. Vorteilhafterweise kann mit diesem Ansatz nicht nur der von dem Herzunterstützungssystem generierte Pumpenvolumenstrom gemessen werden, sondern es kann zudem auch eine Restauswurfleistung des Herzens berücksichtigt werden, um das gesamte Herzzeitvolumen des Patienten bestimmen zu können. Der hier vorgestellte Ansatz kann zudem vorteilhafterweise in Verbindung mit einem Herzunterstützungssystem zum minimalinvasiven Einführen als vollimplantiertes System verwendet werden.

Es wird eine Vorrichtung zum Bestimmen eines Herzzeitvolumens für ein Herzunterstützungssystem vorgestellt. Die Vorrichtung umfasst eine Stützstruktur und eine Sensoreinrichtung auf. Die Stützstruktur weist zumindest eine Strebe und einen Anbindungsabschnitt zum Anbinden der Vorrichtung an ein Element des Herzunterstützungssystems auf. Die zumindest eine Strebe ist mit dem Anbindungsabschnitt verbunden und von dem Element weg bewegbar bzw. wegklappbar. Die Sensoreinrichtung ist mit der Stützstruktur gekoppelt und ausgebildet, um einen Blutstrom zu sensieren.

Das Herzzeitvolumen ist das Volumen des Blutes, das pro Zeitspanne vom Herzen und im Fall eines Herzunterstützungssystems zusätzlich oder alternativ vom Herzunterstützungssystem gepumpt wird. Bei dem Herzunterstützungssystem kann es sich um eine Herzpumpe wie ein ventrikuläres Unterstützungssystem handeln, beispielsweise um ein linksventrikuläres Unterstützungssystem (LVAD, Left Ventricular Assist Device). Die Stützstruktur kann beispielsweise als Trägerstruktur für die Sensoreinrichtung ausgeformt sein. Zusätzlich oder alternativ kann die Stützstruktur auch als Stent, also als zylinderförmige Spiraldrahtprothese zur Gefäßstütze, ausgeformt sein, um die Position des Herzunterstützungssystems in einem Blutgefäß zu stützen. Die zumindest eine Strebe kann beispielsweise der Träger der Sensoreinrichtung sein. Zusätzlich oder alternativ kann die Strebe als Verbindungselement für den Stent verwendet werden. Der Anbindungsabschnitt kann die Vorrichtung beispielsweise formschlüssig oder kraftschlüssig mit dem Element des Herzunterstützungssystems, beispielsweise einem Gehäuse oder einem Leitabschnitt, verbinden. Die Sensoreinrichtung kann den Blutstrom beispielsweise mittels thermischer Anemometrie oder mittels Laser-Doppler-Velocimetrie oder mittels eines Ultraschallelements sensieren. Wenn die Vorrichtung beispielsweise in einer menschlichen Aorta positioniert ist, kann die Sensoreinrichtung ausgeformt sein, um zum Bestimmen des gesamten Herzzeitvolumens als Blutstrom einen aus dem Herzunterstützungssystem ausgeleiteten Pumpenvolumenstrom und eine Restauswurfleistung des Herzens, beispielsweise eine Bypassströmung durch die Aortenklappe hindurch am Herzunterstützungssystem vorbei, zu sensieren. Die Sensoreinrichtung kann einstückig oder mehrstückig ausgeführt sein. Beispielsweise kann die Sensoreinrichtung als integriertes Sensormodul in Form eines Halbleiterchips realisiert sein.

Gemäß einer Ausführungsform kann die Stützstruktur als Verankerungsstruktur zum Verankern des Herzunterstützungssystems in einem Blutgefäß ausgeformt sein. Die Stützstruktur kann beispielsweise ausgebildet sein, um die Vorrichtung und damit das mit der Vorrichtung verbundene Herzunterstützungssystem kraftschlüssig in dem Blutgefäß zu verankern. Als Verankerungsstruktur kann die Stützstruktur dazu beispielsweise eine Komponente mit einem Innendurchmesser aufweisen, der geringfügig größer als der Innendurchmesser des Blutgefäßes ist, in dem die Verankerungsstruktur verankert wird. Beispielsweise kann die Stützstruktur zumindest zwei bogenförmige Streben umfassen, die im weggeklappten Zustand einen solchen Innendurchmesser aufweisen und das Herzunterstützungssystem verankern können, oder die Stützstruktur kann als Stent ausgeformt sein. Auf diese Weise kann die Verankerungsstruktur vorteilhafterweise ein Verrutschen oder eine Dislokation des Herzunterstützungssystems verhindern. Durch die Ausformung der Stützstruktur als Verankerungsstruktur kann vorteilhafterweise auf ein zusätzliches Bauteil zum Verankern des Herzunterstützungssystems verzichtet werden, was eine kompakte Bauweise ermöglicht.

Wenn die Stützstruktur als Verankerungsstruktur ausgeformt ist, kann sie gemäß einer Ausführungsform zudem einen Haltekranz aufweisen, der mit der zumindest einen Strebe verbunden und dazu ausgeformt ist, das Herzunterstützungssystem kraftschlüssig in dem Blutgefäß zu fixieren. Der Haltekranz kann insbesondere zumindest ein Fußteil bzw. Füßchen zum Positionieren der Stützstruktur umfassen. Der Haltekranz kann beispielsweise zusammenfaltbar sein und zum Verankern entfaltbar sein. Im entfalteten Zustand kann der Haltekranz zylinderförmig oder konusförmig sein. Zudem kann der Haltekranz entfaltet eine der nicht exakt kreisrunden Aorten-Anatomie einer menschlichen Aorta entsprechende Form aufweisen. Auf diese Weise kann die Vorrichtung im Verankerungszustand über einen radialen Kraftschluss am Bestimmungsort gehalten werden. Das Fußteil kann beispielsweise eine atraumatische Form aufweisen, um das Blutgefäß nicht zu verletzen. Der Haltekranz kann beispielsweise auch drei Fußteile aufweisen, die zum Positionieren der Fußteile in je einer Kuspe einer Herzklappe ausgeformt sind. Diese Ausführungsform ist von Vorteil, um die Vorrichtung und damit das Herzunterstützungssystem besonders exakt positionieren zu können, insbesondere wenn das Herzunterstützungssystem mittels der Vorrichtung beispielsweise hinter der Aortenklappe positioniert und verankert wird.

Zumindest die Stützstruktur kann gemäß einer Ausführungsform zum Einnehmen eines Einführzustands des Herzunterstützungssystems zusammenfaltbar und zum Einnehmen eines Verankerungszustands des Herzunterstützungssystems entfaltbar sein. Diese Ausführungsform ist vorteilhaft, wenn die Vorrichtung mit dem Herzunterstützungssystem verbunden ist, zum minimalinvasiven Einführen des Herzunterstützungssystems, beispielsweise über einen transfemoralen Zugang. Gemäß dieser Ausführungsform kann die Stützstruktur beispielsweise zylinderförmig zusammenfaltbar sein, und im Einführzustand an einem Abschnitt des Herzunterstützungssystems anliegen. Die Stützstruktur kann beispielsweise so zusammengefaltet sein, dass die Vorrichtung mit dem Herzunterstützungssystem zum minimalinvasiven Einbringen in einen Katheter eingeführt werden kann. Im Einführzustand kann die Vorrichtung entsprechend einen Durchmesser aufweisen, der unter dem Durchmesser einer menschlichen Aorta liegt. Unter dem Verankerungszustand kann ein Zustand verstanden werden, in dem die Stützstruktur, beispielsweise als Verankerungsstruktur, nach dem Einführen und Ausrichten am Bestimmungsort entfaltet ist, um durch ein Vergrößern des Durchmessers der Stützstruktur das Herzunterstützungssystem kraftschlüssig am Bestimmungsort zu verankern.

Zudem kann die Stützstruktur gemäß einer Ausführungsform ein Formgedächtniselement umfassen. Die zumindest eine Strebe kann beispielsweise als Formgedächtniselement ausgeführt sein, was vorteilhafterweise eine einfache Realisierung des Wegklappens der Strebe vom Gehäuse des Herzunterstützungssystems ermöglicht. Zudem kann auch die gesamte Stützstruktur als Formgedächtniselement ausgeformt sein. Das Formgedächtniselement kann beispielsweise aus einem biokompatiblen Formgedächtnispolymer bestehen, oder aus einer biokompatiblen Formgedächtnislegierung, wie beispielsweise Nitinol. Die Verwendung von Nitinol als Formgedächtnismaterial ist vorteilhaft, da der Nitinolwerkstoff in der Medizin, insbesondere im Bereich der kardiovaskulären Medizin, ein bewährtes Material darstellt, beispielsweise für Herzklappenprothesen, Stents und Gefäßprothesen, aufgrund seiner Biokompatibilität und der Formgedächtniseigenschaft, die es ermöglicht auch komplexe Strukturen auf kleinem Bauraum an den Bestimmungsort zu liefern und abzusetzen.

Die zumindest eine Strebe kann gemäß einer Ausführungsform eine Sensorplattform aufweisen. Zumindest ein Sensorelement der Sensoreinrichtung kann auf der Sensorplattform angeordnet sein. Die Strebe kann beispielsweise stabförmig sein und als Sensorplattform eine runde oder ovale Ausbuchtung aufweisen, oder die Sensorplattform kann als runde oder ovale Vertiefung ausgeformt sein. Die Ausformung einer Sensorplattform auf, in oder an der zumindest einen Strebe ermöglicht vorteilhafterweise eine platzsparende Integration des Sensorelements der Sensoreinrichtung und eine kompakte Bauweise der Stützstruktur.

Ferner kann die zumindest eine Strebe gemäß einer Ausführungsform eine Leitungseinrichtung aufweisen. Die Leitungseinrichtung kann ausgebildet sein, zumindest ein Sensorelement der Sensoreinrichtung elektrisch zu kontaktieren. Die Leitungseinrichtung kann beispielsweise entlang der Strebe geführt sein. Die Leitungseinrichtung kann auch elektrische Kontaktpads aufweisen, die über elektrische Leiterbahnen kontaktiert sein können. Optional können die Kontaktpads auf der Sensorplattform angeordnet sein. Die Leitungseinrichtung kann auch durch eine elektrische Funktionalisierung zumindest eines Teils der Stützstruktur realisiert sein, beispielsweise wenn der Teil der Stützstruktur als Formgedächtniselement aus Nitinol ausgeformt ist. Zusätzlich oder alternativ kann die Leitungseinrichtung ein entsprechend ausgeformtes Dünnschichtsubstrat als elektrische Leitung aufweisen.

Außerdem kann die Sensoreinrichtung zum Sensieren des Blutstroms gemäß einer Ausführungsform eine Ultraschalleinrichtung oder eine Anemometrie-Einrichtung umfassen. Wenn die Sensoreinrichtung eine Ultraschalleinrichtung umfasst, kann die Sensoreinrichtung beispielsweise einen Ultraschallwandler, beispielsweise ein bidirektionales Ultraschallelement als Sender und Empfänger aufweisen. Der Blutstrom kann in diesem Fall mittels Doppler-Ultraschallmesstechnik sensiert werden. Die Messung kann dabei beispielsweise innerhalb der Vorrichtung an einer Stelle erfolgen, an dem im implantierten Zustand der Vorrichtung keine Verwirbelungen aufgrund der zumindest einen Strebe im Blutstrom auftreten. Wenn die Sensoreinrichtung eine Anemometrie-Einrichtung umfasst, kann das Sensieren des Blutstroms beispielsweise mittels thermischer Anemometrie oder mittels Laser-Doppler-Anemometrie (Laser-Doppler-Velocimetrie) erfolgen.

Wenn die Sensoreinrichtung gemäß einer Ausführungsform die Anemometrie-Einrichtung aufweist, kann die Anemometrie-Einrichtung ein Heizelement und einen Temperatursensor umfassen. Alternativ kann die Anemometrie-Einrichtung eine Lichtquelle und eine Photodiode umfassen. Das Heizelement kann beispielsweise ein Heizfilament sein. Der Temperatursensor kann beispielsweise ein Referenz-Bluttemperatursensor sein. Zudem kann der Temperatursensor auch an der Spitze des Herzunterstützungssystems angeordnet sein, beispielsweise wenn die Kopfeinheit an der Spitze des Herzunterstützungssystems eine Sensorbaugruppe mit einem Temperatursensor umfasst. Eine Auswerteeinheit der Anemometrie-Einrichtung kann beispielsweise die Energieabfuhr des Blutes, beispielsweise an einem beheizten zweiten Temperatursensor, erfassen. Das Heizelement kann dabei beispielsweise als zweiter Temperatursensor verwendet werden, oder das Heizelement kann dazu thermisch mit einem zweiten Temperatursensor verbunden sein. Das Heizelement kann beispielsweise an der zumindest einen Strebe angeordnet sein, und zusätzlich oder alternativ kann der zweite Temperatursensor auf oder unter oder in dem Heizelement auf der Strebe angeordnet sein. Wenn die Anemometrie-Einrichtung eine Lichtquelle und eine Photodiode umfasst, kann die Lichtquelle ein Laser, beispielsweise ein sogenannter Vertical Cavity Surface Emitting Laser, sein. Aufgrund der optionalen Anordnung der Sensoreinrichtung mitten in der Strömung des Blutstroms ist das optische Messfenster vorteilhafterweise zusätzlich vor Gewebeablagerungen geschützt.

Es wird ferner ein Herzunterstützungssystem vorgestellt. Das Herzunterstützungssystem weist eine Kopfeinheit, einen an die Kopfeinheit angrenzenden Einlassabschnitt zum Einleiten eines Blutstroms, einen an den Einlassabschnitt angrenzenden Leitabschnitt zum Leiten des Blutstroms, einen an den Leitabschnitt angrenzenden Auslassabschnitt zum Ausleiten des Blutstroms, ein an den Auslassabschnitt angrenzendes Gehäuse, eine in dem Gehäuse angeordnete Antriebseinrichtung und ein Zuleitungskabel zum elektrischen Kontaktieren des Herzunterstützungssystems auf, sowie eine Ausführungsform der vorstehend vorgestellten Vorrichtung zum Bestimmen eines Herzzeitvolumens für ein Herzunterstützungssystem. Das Herzunterstützungssystem kann beispielsweise als linksventrikuläres Herzunterstützungssystem ausgeformt sein. Das Herzunterstützungssystem kann beispielsweise zum Positionieren des Herzunterstützungssystems im Bereich einer menschlichen Aorta ausgeformt sein. Vorteilhafterweise kann das Herzunterstützungssystem somit für ein Bestimmen des Herzzeitvolumens in einem vollimplantierten Herzunterstützungssystem verwendet werden. Dies ist von Vorteil, um beim Betreiben des Herzunterstützungssystems im implantierten Zustand beispielsweise ein kontinuierliches Messen des Herzzeitvolumens zur Überwachung und Unterstützung des menschlichen Kreislaufs durch das Herzunterstützungssystems ermöglichen zu können.

Gemäß einer Ausführungsform des Herzunterstützungssystems kann zumindest ein Sensorelement der Sensoreinrichtung der Vorrichtung in der Kopfeinheit des Herzunterstützungssystems und zumindest ein weiteres Sensorelement der Sensoreinrichtung in der Stützstruktur der Vorrichtung angeordnet sein. Wenn die Sensoreinrichtung beispielsweise eine Anemometrie-Einrichtung mit einem Heizelement und einem Temperatursensor umfasst, kann der Temperatursensor beispielsweise in der Kopfeinheit des Herzunterstützungssystems und das Heizelement in der Stützstruktur der Vorrichtung angeordnet sein. Diese Anordnung ermöglicht vorteilhafterweise eine kompakte Bauweise. Alternativ können das Sensorelement und das weitere Sensorelement auf unterschiedlichen Streben angeordnet sein. Beispielsweise kann ein Anemometrie-Sensor auf einer Strebe und ein Referenzsensor auf einer anderen Strebe angeordnet sein.

Durch ein Verwenden einer vorstehend genannten Ausführungsform der Vorrichtung kann ein Herzzeitvolumen vorteilhaft bestimmt werden.

Es wird ein Verfahren zum Bestimmen eines Herzzeitvolumens unter Verwendung einer vorstehend genannten Ausführungsform der Vorrichtung vorgestellt. Das Verfahren umfasst folgende Schritte:
Bereitstellen eines Steuersignals, wobei das Steuersignal ausgebildet ist, die Sensoreinrichtung zum Aussenden eines Anregungssignals zum Sensieren des Blutstroms anzusteuern;
Empfangen eines Sensorsignals, das von der Sensoreinrichtung bereitgestellt wird; und
Bestimmen des Herzzeitvolumens unter Verwendung des Sensorsignals.

Die Schritte des Verfahrens können beispielsweise unter Verwendung einer Steuereinrichtung der Vorrichtung ausgeführt werden. Unter einer Steuereinrichtung kann vorliegend eine elektrische Schaltung verstanden werden, die Sensorsignale verarbeitet und in Abhängigkeit davon Steuer- und/oder Datensignale ausgibt. Die Steuereinrichtung kann separat zu der Sensoreinrichtung ausgeführt sein oder in der Sensoreinrichtung integriert sein.

Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung eines Herzunterstützungssystems mit einer Vorrichtung zum Bestimmen eines Herzzeitvolumens gemäß einem Ausführungsbeispiel;
- Fig. 2: eine schematische Darstellung eines Herzunterstützungssystems mit einer Vorrichtung zum Bestimmen eines Herzzeitvolumens gemäß einem Ausführungsbeispiel;
- Fig. 3: eine schematische Darstellung einer Stützstruktur einer Vorrichtung zum Bestimmen eines Herzzeitvolumens gemäß einem Ausführungsbeispiel;
- Fig. 4: eine schematische Darstellung eines Teils einer Stützstruktur einer Vorrichtung zum Bestimmen eines Herzzeitvolumens gemäß einem Ausführungsbeispiel; und
- Fig. 5: ein Ablaufdiagramm eines Verfahrens zum Bestimmen eines Herzzeitvolumens gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

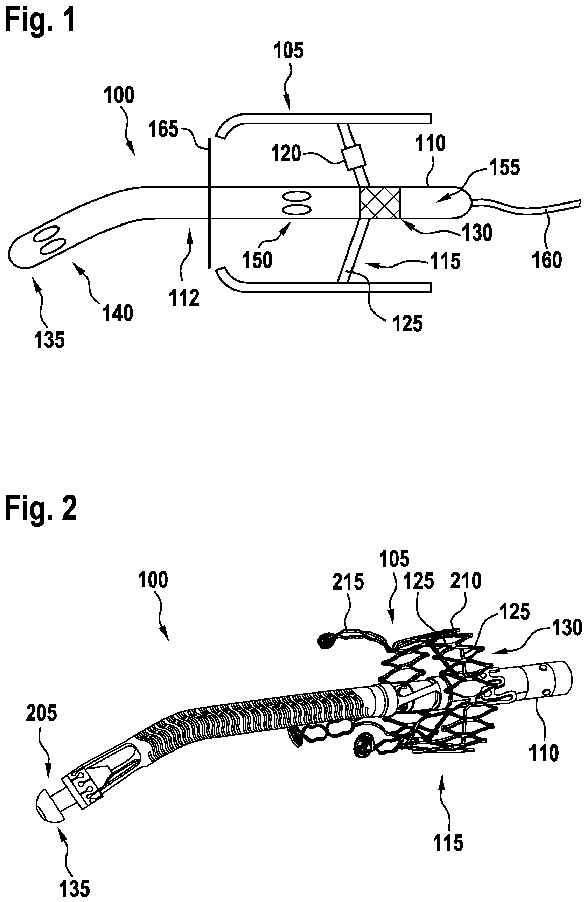

Fig. 1 zeigt eine schematische Darstellung eines Herzunterstützungssystems 100 mit einer Vorrichtung 105 zum Bestimmen eines Herzzeitvolumens gemäß einem Ausführungsbeispiel. Gezeigt ist eine Seitenansicht des Herzunterstützungssystems 100, das beispielhaft als linksventrikuläres Herzunterstützungssystem ausgeführt ist und eine längliche Form aufweist. Die Vorrichtung 105 ist mit einem Element des Herzunterstützungssystems verbunden. Beispielhaft ist das Element hier als Abschnitt eines Gehäuses 110 des Herzunterstützungssystems 100 ausgeführt, alternativ kann das Element auch ein Leitabschnitt 112 des Herzunterstützungssystems 100 sein.

Die Vorrichtung 105 weist gemäß einem Ausführungsbeispiel eine Stützstruktur 115 und eine Sensoreinrichtung 120 auf. Die Stützstruktur 115 weist zumindest eine Strebe 125 und einen Anbindungsabschnitt 130 zum Anbinden der Vorrichtung 105 an ein Element 110 des Herzunterstützungssystems 100 auf. Die zumindest eine Strebe 125 ist mit dem Anbindungsabschnitt 130 verbunden und von dem Element 110 wegklappbar. Die Sensoreinrichtung 120 ist mit der zumindest einen Strebe 125 gekoppelt und ausgebildet, um einen Blutstrom zu sensieren.

Gemäß einem Ausführungsbeispiel weist das Herzunterstützungssystem 100 eine Kopfeinheit 135, einen an die Kopfeinheit 135 angrenzenden Einlassabschnitt 140 zum Einleiten eines Blutstroms, den an den Einlassabschnitt 140 angrenzenden Leitabschnitt 112 zum Leiten des Blutstroms, einen an den Leitabschnitt 112 angrenzenden Auslassabschnitt 150 zum Ausleiten des Blutstroms, ein an den Auslassabschnitt 150 angrenzendes Gehäuse, eine in dem Gehäuse angeordnete Antriebseinrichtung 155 und ein Zuleitungskabel 160 zum elektrischen Kontaktieren des Herzunterstützungssystems 100 auf, sowie die Vorrichtung 105.

Wenn das Herzunterstützungssystem 100 als linksventrikuläres Herzunterstützungssystem in einer menschlichen Aorta implantiert ist, ist der Einlassabschnitt 140 in einem linken Ventrikel angeordnet. Dort wird das Blut aus dem Ventrikel angesaugt, durch den Leitabschnitt 112 geleitet und über die Auslassöffnungen des Auslassabschnitts 150 in der Aorta ausgeworfen. Die Linie 165 kennzeichnet die Klappenebene einer Aortenklappe. Im implantierten Zustand des Herzunterstützungssystems 100 liegen in dem durch die Linie 165 markierten Bereich die Aortenklappen und trennen den Aorten- und den Ventrikelbereich. Zum Fixieren der beschriebenen Position des Herzunterstützungssystems 100 im implantierten Zustand weist die Vorrichtung 105 als Stützstruktur 115 optional eine Verankerungsstruktur auf, wie anhand der folgenden Fig. 2 beschrieben.

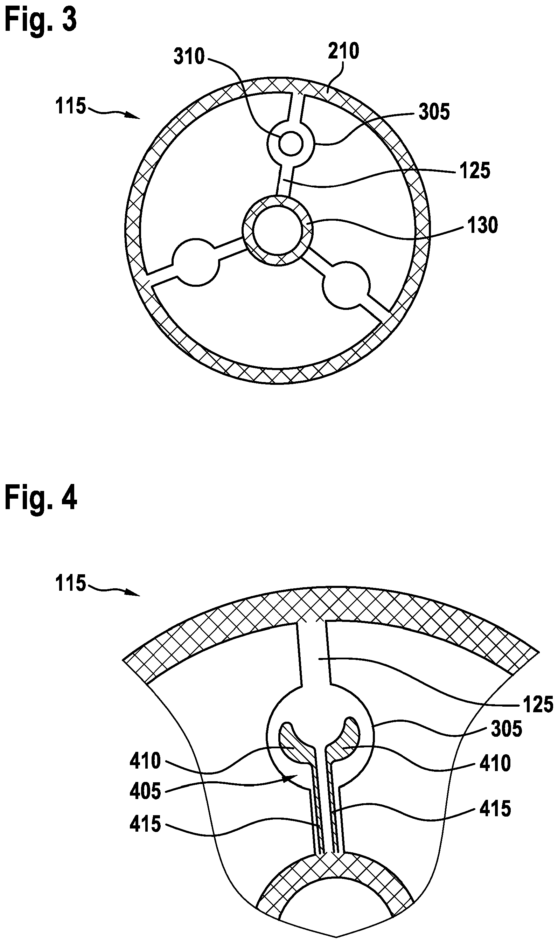

Die Sensoreinrichtung 120 umfasst gemäß einem Ausführungsbeispiel zum Sensieren des Blutstroms eine Ultraschalleinrichtung oder eine Anemometrie-Einrichtung. Optional umfasst die Anemometrie-Einrichtung ein Heizelement und einen Temperatursensor oder die Anemometrie-Einrichtung umfasst eine Lichtquelle und eine Photodiode. Die genannten Elemente sind optional in der Stützstruktur 115 angeordnet, beispielsweise an der zumindest einen Strebe 125, wie anhand von Fig. 4 beschrieben.

Fig. 2 zeigt eine schematische Darstellung eines Herzunterstützungssystems 100 mit einer Vorrichtung 105 zum Bestimmen eines Herzzeitvolumens gemäß einem Ausführungsbeispiel. Es ist eine Seitenansicht des Herzunterstützungssystems 100 gezeigt, das im Wesentlichen dem in der vorstehenden Fig. 1 beschriebenen Herzunterstützungssystem entspricht, mit Ausnahme des hier nicht gezeigten Zuleitungskabels und der Ausformung der Stützstruktur 115 und der Kopfeinheit 135. Das Herzunterstützungssystem 100 ist hier beispielhaft als linksventrikuläres Herzunterstützungssystem 100 für die Aortenklappenposition gezeigt. Die Stützstruktur 115 ist gemäß dem hier gezeigten Ausführungsbeispiel als Verankerungsstruktur 115 zum Verankern des Herzunterstützungssystems 100 in einem Blutgefäß ausgeformt, und ist entsprechend mit dem gleichen Bezugszeichen 115 versehen. Die Verankerungsstruktur 115 kann auch als Stent bezeichnet werden und beim Implantieren des Herzunterstützungssystems 100 auch zum Positionieren und Fixieren des Herzunterstützungssystems 100 im Bereich der Aorta verwendet werden.

Die Stützstruktur 115 umfasst gemäß einem Ausführungsbeispiel ein Formgedächtniselement. Beispielhaft ist hier die Stützstruktur 115 als Verankerungsstruktur 115 aus Nitinol, einer hochelastischen Nickel-Titan-Formgedächtnislegierung, ausgeformt. Als Anbindungsabschnitt 130 weist die Verankerungsstruktur 115 hier einen lasergeschnittener Clip auf, der die Verankerungsstruktur 115 auf dem Gehäuse 110 im Bereich des Antriebs des Herzunterstützungssystems fixiert.

Die Kopfeinheit 135 weist hier eine Sensorbaugruppe 205 auf. Gemäß einem Ausführungsbeispiel ist zumindest ein Sensorelement der anhand von Fig. 1 beschriebenen Sensoreinrichtung der Vorrichtung 105 in der Kopfeinheit 135 des Herzunterstützungssystems 100 angeordnet, beispielsweise als Teil der Sensorbaugruppe 205. Zumindest ein weiteres Sensorelement der Sensoreinrichtung ist in der Stützstruktur 115 der Vorrichtung angeordnet. Alternativ ist die gesamte Sensoreinrichtung in der Stützstruktur 115 angeordnet.

Die Verankerungsstruktur 115 umfasst gemäß dem hier gezeigten Ausführungsbeispiel einen Haltekranz 210. Der Haltekranz 210 ist mit der zumindest einen Strebe 125 verbunden und dazu ausgeformt, das Herzunterstützungssystem 100 kraftschlüssig in dem Blutgefäß zu fixieren. Der Haltekranz 210 weist insbesondere zumindest ein Füßchen 215 zum Positionieren der Verankerungsstruktur 115 auf. Beispielhaft sind hier zwei bogenförmig gekrümmte Füßchen 215 gezeigt. Die Verankerungsstruktur 115 ist optional zum Einnehmen eines Einführzustands des Herzunterstützungssystems 100 zusammenfaltbar und zum Einnehmen eines Verankerungszustands des Herzunterstützungssystems 100 entfaltbar. Hier ist die Verankerungsstruktur 115 beispielhaft entfaltet gezeigt, entsprechend einem Verankerungszustand, wenn das Herzunterstützungssystem 100 in einem Blutgefäß, beispielsweise in der Aorta, verankert ist. Die Verankerungsstruktur 115 weist hier beispielhaft eine Mehrzahl an gleichmäßig beabstandeten Streben 125 auf, die von dem Gehäuse 110 des Herzunterstützungssystems 100 weggeklappt sind, entsprechend dem entfalteten Zustand der Verankerungsstruktur 115. Die zumindest eine Strebe 125 ist dazu optional als Formgedächtniselement ausgeführt und weist eine voreingeprägte Form auf, um einen Federdruck nach außen aufzubringen und somit den Haltekranz 210 gegen die Gefäßwand des Blutgefäßes zu drücken. Der Haltekranz 210 weist optional ebenfalls eine voreingeprägte Form auf, um im Verankerungszustand eine Federkraft gegen die Aortenwand aufzubringen. Darüber hinaus weist die Verankerungsstruktur optional drei Füßchen 215 auf, die in den Taschen oberhalb der Aortenklappen positionierbar sind und die Position des Herzunterstützungssystems 100, im Folgenden auch Pumpe genannt, weiter fixieren.

Die hier gezeigte Verankerungsstruktur 115 weist gemäß einem Ausführungsbeispiel die Sensoreinrichtung als integrierte Messtechnik auf. Zumindest ein Sensorelement der Sensoreinrichtung ist optional an der zumindest einen Strebe 125 angeordnet und befindet sich in diesem Fall vorteilhafterweise im Blutstrom des Gesamt-Herzzeitvolumens in der Aorta, um den Blutstrom zu sensieren, wenn das Herzunterstützungssystems 100 implantiert ist .

Geeignete Messverfahren der Sensoreinrichtung sind beispielsweise Heizfilament-Anemometrie oder Doppler-Ultraschall, wie anhand der folgenden Figuren 3 und 4 beschrieben.

Fig. 3 zeigt eine schematische Darstellung einer Stützstruktur 115 einer Vorrichtung zum Bestimmen eines Herzzeitvolumens gemäß einem Ausführungsbeispiel. Es ist eine Aufsicht auf einen Querschnitt der Stützstruktur 115 gezeigt. Die hier dargestellte Stützstruktur ähnelt oder entspricht der Stützstruktur aus der vorstehend beschriebenen Fig. 2, mit Ausnahme der Ausformung der zumindest einen Strebe 125, und weist den Haltekranz 210 und den Anbindungsabschnitt 130 auf. Die Stützstruktur 115 weist hier beispielhaft drei Streben 125 auf.

Gemäß dem hier gezeigten Ausführungsbeispiel weist jede Strebe 125 eine Sensorplattform 305 auf. Zumindest ein Sensorelement 310 der anhand von Fig. 1 beschriebenen Sensoreinrichtung ist auf der Sensorplattform 305 angeordnet. Alternativ ist die gesamte Sensoreinrichtung auf der Sensorplattform 305 angeordnet. Die Integration von Sensorik entlang der Strebe 125, beispielsweise auf der Sensorplattform 305, ist vorteilhaft im Hinblick auf eine kompakte Bauweise. Als Sensorelement 310 der Sensoreinrichtung kann beispielsweise ein Ultraschallwandler oder ein Sensor einer Anemometrie-Einrichtung wie anhand der folgenden Fig. 4 beschrieben, auf der Sensorplattform 305 angeordnet sein. Die Sensorplattform 305 weist hier beispielhaft eine runde Form auf und ist in Bezug auf den Abstand des Anbindungsabschnitts 130 von dem Haltekranz 210 etwa auf der Hälfte der Längserstreckung der Strebe 125 als Ausbuchtung der Strebe 125 ausgeformt.

Fig. 4 zeigt eine schematische Darstellung eines Teils einer Stützstruktur 115 einer Vorrichtung zum Bestimmen eines Herzzeitvolumens gemäß einem Ausführungsbeispiel. Gezeigt ist ein Ausschnitt der Stützstruktur 115, wie sie anhand von Fig. 3 beschrieben ist, wobei die Strebe 125 gemäß dem hier gezeigten Ausführungsbeispiel zusätzlich eine Leitungseinrichtung 405 aufweist.

Die Leitungseinrichtung 405 ist ausgebildet, zumindest ein Sensorelement der Sensoreinrichtung elektrisch zu kontaktieren. Die Leitungseinrichtung kann beispielsweise ausgeformt sein, um als Sensorelement der Sensoreinrichtung den Ultraschallwandler elektrisch zu kontaktieren. Beispielsweise kann das Sensorelement oder die gesamte Sensoreinrichtung über die Leitungseinrichtung 405 mit elektrischer Energie versorgt werden und zusätzlich oder alternativ mit einer Steuereinrichtung oder einer Kommunikationseinrichtung zur Datenübertragung verbunden werden.

Die Leitungseinrichtung 405 ist hier beispielhaft als zweipolige elektrische Leistungszuführung gezeigt, und weist zwei auf der Sensorplattform 305 integrierte Kontaktpads 410 auf, die über elektrische Leiterbahnen 415 kontaktiert sind. Die Realisierung der elektrischen Leiterstruktur erfolgt optional durch eine elektrische Funktionalisierung der gemäß einem Ausführungsbeispiel als Formgedächtniselement aus Nitinol ausgeformten Stützstruktur 115. Alternativ erfolgt die elektrische Funktionalisierung durch Aufbringen einer separaten, in der Form der Stützstruktur 115 angepassten Leiterstruktur, beispielsweise durch ein Dünnschichtsubstrat als elektrische Leitung.

Zum Bestimmen des Herzzeitvolumens wird vorteilhafterweise ein vorstehend genanntes Ausführungsbeispiel der Vorrichtung verwendet, wobei das Messen der Blutflussgeschwindigkeit je nach Ausführungsbeispiel mittels thermischer Anemometrie, Laser-Doppler-Velocimetrie oder Doppler-Ultraschall erfolgt. Die Sensoreinrichtung umfasst gemäß einem Ausführungsbeispiel eine Anemometrie-Einrichtung, die optional ein Heizelement und einen Temperatursensor umfasst. Als Heizelement kann mindestens ein Heizfilament entlang der Strebe 125 integriert sein, beispielsweise auf der Sensorplattform 305. Heizelement und Temperatursensor können gemeinsam auf einer Strebe 125 oder auf unterschiedlichen Streben 125 angeordnet sein, sodass der Temperatursensor auf einer Strebe 125 und das Heizelement auf einer anderen Strebe 125 sitzen kann. Somit kann die Sensoreinrichtung über mehrere Streben 125 verteilt sein. Zusätzlich ist optional als Temperatursensor ein Referenz-Bluttemperatursensor auf der Sensorplattform 305 integriert. Ein solcher Referenz-Bluttemperatursensor kann auch auf einer anderen Strebe 125 angeordnet sein. Alternativ ist der Temperatursensor in der Spitze des Herzunterstützungssystems in der Kopfeinheit positioniert. Eine Auswerteeinheit erfasst dabei die Energieabfuhr (vergleichbar mit einem Kühlmittelstrom) des Blutes an einem beheizten Anemometrie-Sensor vorbei. Das Heizfilament ist optional als zweiter Temperatursensor ausgeformt oder ein weiterer Temperatursensor ist thermisch mit dem Heizfilament verbunden und auf, unter, oder im Heizfilament auf der Strebe 125 angeordnet.

Zum Bestimmen des Herzzeitvolumens mittels Laser-Doppler-Velocimetrie umfasst die Anemometrie-Einrichtung eine Lichtquelle wie einen Laser und eine Photodiode. Die Lichtquelle und die Photodiode sind optional auf einer oder mehrerer Sensorplattformen 305 integriert. Dies ist vorteilhafterweise platzsparend und ermöglicht, die Messtechnik thermisch vom Antrieb zu entkoppeln.

Alternativ umfasst die Sensoreinrichtung zum Bestimmen des Herzzeitvolumens mittels Doppler-Ultraschall eine Ultraschalleinrichtung, die zur Flussmessung geeignet ist. Dabei werden von einem Ultraschallwandler Schallimpulse ausgesendet und der Phasenfortschritt des zurückgestreuten Schallimpulses analysiert. Das Pulsed-Wave-Doppler-Verfahren ermöglicht dabei eine gezielte Wahl der Betrachtungstiefe, beispielsweise erfolgt die Messung weiter weg von der Strebe 125, sodass keine Turbulenzen oder Verwirbelungen aufgrund der Strebe 125 nachweisbar sind. Die Ultraschalleinrichtung umfasst optional ein bidirektionales Ultraschallelement für die Sende- und Empfangsrichtung. In diesem Fall ist als Sensorelement der Sensoreinrichtung der Ultraschallwandler oder das bidirektionale Ultraschallelement an der Stützeinrichtung angeordnet, und optional auf der Strebe 125 oder der Sensorplattform 305 integriert.

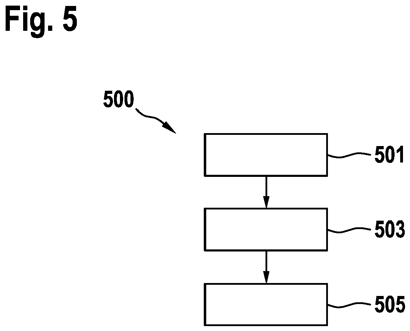

Fig. 5 zeigt ein Ablaufdiagramm eines Verfahrens 500 zum Bestimmen eines Herzzeitvolumens unter Verwendung eines Ausführungsbeispiels der vorstehend genannten Vorrichtung. Das Verfahren 500 weist einen Schritt 501 des Bereitstellens, eine Schritt 503 des Empfangens und einen Schritt 505 des Bestimmens auf. Im Schritt 501 des Bereitstellens wird ein Steuersignal bereitgestellt, das ausgebildet ist, die Sensoreinrichtung, wie sie beispielsweise anhand von Fig. 1 beschrieben ist, zum Aussenden eines Anregungssignals zum Sensieren des Blutstroms anzusteuern. Im Schritt 503 des Empfangens wird ein Sensorsignal, das von der Sensoreinrichtung bereitgestellt wird, empfangen. Im Schritt 505 des Bestimmens wird das Herzzeitvolumen unter Verwendung des Sensorsignals bestimmt.

## Patentansprüche

1. Vorrichtung (105) zum Bestimmen eines Herzzeitvolumens für ein Herzunterstützungssystem (100), wobei die Vorrichtung (105) folgende Merkmale aufweist:
eine Stützstruktur (115), die zumindest eine Strebe (125) und einen Anbindungsabschnitt (130) zum Anbinden der Vorrichtung (105) an ein Element (110, 112) des Herzunterstützungssystems (100) aufweist, wobei die zumindest eine Strebe (125) mit dem Anbindungsabschnitt (130) verbunden und von dem Element (110, 112) weg bewegbar, insbesondere wegklappbar ist; und
eine Sensoreinrichtung (120), die mit der zumindest einen Strebe (125) gekoppelt und ausgebildet ist, um einen Blutstrom zu sensieren.

2. Vorrichtung (105) gemäß Anspruch 1, wobei die Stützstruktur (115) als Verankerungsstruktur (115) zum Verankern des Herzunterstützungssystems (100) in einem Blutgefäß ausgeformt ist.

3. Vorrichtung (105) gemäß Anspruch 2, wobei die Stützstruktur (115) einen Haltekranz (210) aufweist, der mit der zumindest einen Strebe (125) verbunden und dazu ausgeformt ist, das Herzunterstützungssystem (100) kraftschlüssig in dem Blutgefäß zu fixieren, insbesondere wobei der Haltekranz (210) zumindest ein Fußteil (215) zum Positionieren der Stützstruktur (115) umfasst.

4. Vorrichtung (105) gemäß einem der vorangegangenen Ansprüche, wobei zumindest die Stützstruktur (115) zum Einnehmen eines Einführzustands des Herzunterstützungssystems (100) zusammenfaltbar und zum Einnehmen eines Verankerungszustands des Herzunterstützungssystems (100) entfaltbar ist.

5. Vorrichtung (105) gemäß einem der vorangegangenen Ansprüche, wobei die Stützstruktur (115) ein Formgedächtniselement umfasst.

6. Vorrichtung (105) gemäß einem der vorangegangenen Ansprüche, wobei die zumindest eine Strebe (125) eine Sensorplattform (305) aufweist, wobei zumindest ein Sensorelement (310) der Sensoreinrichtung (120) auf der Sensorplattform (305) angeordnet ist.

7. Vorrichtung (105) gemäß einem der vorangegangenen Ansprüche, wobei die zumindest eine Strebe (125) eine Leitungseinrichtung (405) aufweist, die ausgebildet ist, zumindest ein Sensorelement (310) der Sensoreinrichtung (120) elektrisch zu kontaktieren.

8. Vorrichtung (105) gemäß einem der vorangegangenen Ansprüche, wobei die Sensoreinrichtung (120) zum Sensieren des Blutstroms eine Ultraschalleinrichtung oder eine Anemometrie-Einrichtung umfasst.

9. Vorrichtung (105) gemäß Anspruch 8, wobei die Anemometrie-Einrichtung ein Heizelement und einen Temperatursensor umfasst, oder wobei die Anemometrie-Einrichtung eine Lichtquelle und eine Photodiode umfasst.

10. Herzunterstützungssystem (100) mit einer Kopfeinheit (135), einem an die Kopfeinheit (135) angrenzenden Einlassabschnitt (140) zum Einleiten eines Blutstroms, einem an den Einlassabschnitt (140) angrenzenden Leitabschnitt (112) zum Leiten des Blutstroms, einem an den Leitabschnitt (112) angrenzenden Auslassabschnitt (150) zum Ausleiten des Blutstroms, einem an den Auslassabschnitt (150) angrenzenden Gehäuse (110), einer in dem Gehäuse (110) angeordneten Antriebseinrichtung (155) und einem Zuleitungskabel (160) zum elektrischen Kontaktieren des Herzunterstützungssystems (100), **dadurch gekennzeichnet, dass** das Herzunterstützungssystem (100) eine Vorrichtung (105) gemäß einem der Ansprüche 1 bis 9 aufweist.

11. Herzunterstützungssystem (100) gemäß Anspruch 10, wobei zumindest ein Sensorelement der Sensoreinrichtung (120) der Vorrichtung (105) in der Kopfeinheit (135) des Herzunterstützungssystems (100) und zumindest ein weiteres Sensorelement (310) der Sensoreinrichtung (120) in der Stützstruktur (115) der Vorrichtung (105) angeordnet sind oder das Sensorelement und das weitere Sensorelement (310) auf unterschiedlichen Streben (125) angeordnet sind.

12. Verwenden einer implantierten Vorrichtung (105) gemäß einem der Ansprüche 1 bis 9 zum Bestimmen eines Herzzeitvolumens.

13. Verfahren (500) zum Bestimmen eines Herzzeitvolumens unter Verwendung einer implantierten Vorrichtung (105) gemäß einem der Ansprüche 1 bis 9, wobei das Verfahren (500) folgende Schritte umfasst:
Bereitstellen (501) eines Steuersignals, wobei das Steuersignal ausgebildet ist, die Sensoreinrichtung (120) zum Aussenden eines Anregungssignals zum Sensieren des Blutstroms anzusteuern;
Empfangen (503) eines Sensorsignals, das von der Sensoreinrichtung (120) bereitgestellt wird; und
Bestimmen (505) des Herzzeitvolumens unter Verwendung des Sensorsignals.

## Claims

1. Device (105) for determining a cardiac output for a cardiac support system (100), wherein the device (105) comprises the following features:
a support structure (115), which has at least one strut (125) and a connection portion (130) for connecting the device (105) to an element (110, 112) of the cardiac support system (100), wherein the at least one strut (125) is connected to the connection portion (130) and can be moved away, in particular folded away, from the element (110, 112); and
a sensor apparatus (120), which is coupled to the at least one strut (125) and is designed to sense a blood flow.

2. Device (105) according to claim 1, wherein the support structure (115) is in the form of an anchoring structure (115) for anchoring the cardiac support system (100) in a blood vessel.

3. Device (105) according to claim 2, wherein the support structure (115) has a retaining ring (210) which is connected to the at least one strut (125) and is formed in order to fix the cardiac support system (100) in the blood vessel in a frictionally-locked manner, in particular wherein the retaining ring (210) comprises at least one foot part (215) for positioning the support structure (115).

4. Device (105) according to any of the preceding claims, wherein at least the support structure (115) can be folded up to assume an insertion state of the cardiac support system (100) and can be unfolded to assume an anchored state of the cardiac support system (100).

5. Device (105) according to any of the preceding claims, wherein the support structure (115) comprises a shape memory element.

6. Device (105) according to any of the preceding claims, wherein the at least one strut (125) has a sensor platform (305), wherein at least one sensor element (310) of the sensor apparatus (120) is arranged on the sensor platform (305).

7. Device (105) according to any of the preceding claims, wherein the at least one strut (125) has a line apparatus (405) which is designed to electrically contact at least one sensor element (310) of the sensor apparatus (120).

8. Device (105) according to any of the preceding claims, wherein the sensor apparatus (120) for sensing the blood flow comprises an ultrasonic apparatus or an anemometry apparatus.

9. Device (105) according to claim 8, wherein the anemometry apparatus comprises a heating element and a temperature sensor, or wherein the anemometry apparatus comprises a light source and a photodiode.

10. Cardiac support system (100) comprising a head unit (135), an inlet portion (140) adjoining the head unit (135) for initiating a blood flow, a conducting portion (112) adjoining the inlet portion (140) for conducting the blood flow, an outlet portion (150) adjacent to the conducting portion (112) for conducting the blood flow out, a housing (110) adjacent to the outlet portion (150), a drive apparatus (155) arranged in the housing (110), and a supply cable (160) for electrically contacting the cardiac support system (100), **characterized in that** the cardiac support system (100) comprises a device (105) according to any of claims 1 to 9.

11. Cardiac support system (100) according to claim 10, wherein at least one sensor element of the sensor apparatus (120) of the device (105) is arranged in the head unit (135) of the heart support system (100) and at least one further sensor element (310) of the sensor apparatus (120) is arranged in the support structure (115) of the device (105), or the sensor element and the further sensor element (310) are arranged on different struts (125).

12. Use of an implanted device (105) according to any of claims 1 to 9 for determining a cardiac output.

13. Method (500) for determining a cardiac output using an implanted device (105) according to any of claims 1 to 9, wherein the method (500) comprises the following steps:
providing (501) a control signal, wherein the control signal is designed to activate the sensor apparatus (120) to emit an excitation signal for sensing the blood flow;
receiving (503) a sensor signal provided by the sensor apparatus (120); and
determining (505) cardiac output using the sensor signal.

## Revendications

1. Dispositif (105) permettant de déterminer un débit cardiaque pour un système d'assistance cardiaque (100), dans lequel le dispositif (105) comporte les caractéristiques suivantes :
une structure de support (115), laquelle comporte au moins une entretoise (125) et une section de liaison (130) permettant de relier le dispositif (105) à un élément (110, 112) du système d'assistance cardiaque (100), dans lequel l'au moins une entretoise (125) est reliée à la section de liaison (130) et peut être éloignée, en particulier repliée, de l'élément (110, 112) ; et
un agencement de capteur (120), lequel est accouplé à l'au moins une entretoise (125) et est conçu pour détecter un flux sanguin.

2. Dispositif (105) selon la revendication 1, dans lequel la structure de support (115) est formée comme une structure d'ancrage (115) permettant d'ancrer le système d'assistance cardiaque (100) dans un vaisseau sanguin.

3. Dispositif (105) selon la revendication 2, dans lequel la structure de support (115) comporte une couronne de retenue (210), laquelle est reliée à l'au moins une entretoise (125) et formée de manière à fixer par liaison par complémentarité de force le système d'assistance cardiaque (100) dans le vaisseau sanguin, en particulier, dans lequel la couronne de retenue (210) comprend au moins une partie pied (215) permettant de positionner la structure de support (115).

4. Dispositif (105) selon l'une quelconque des revendications précédentes, dans lequel au moins la structure de support (115) peut être repliée pour prendre un état d'insertion du système d'assistance cardiaque (100) et peut être dépliée pour prendre un état d'ancrage du système d'assistance cardiaque (100).

5. Dispositif (105) selon l'une quelconque des revendications précédentes, dans lequel la structure de support (115) comprend un élément à mémoire de forme.

6. Dispositif (105) selon l'une quelconque des revendications précédentes, dans lequel l'au moins une entretoise (125) comporte une plate-forme de capteur (305), dans lequel au moins un élément capteur (310) de l'agencement de capteur (120) est disposé sur la plate-forme de capteur (305).

7. Dispositif (105) selon l'une quelconque des revendications précédentes, dans lequel l'au moins une entretoise (125) comporte un agencement de conduite (405), lequel est conçu pour établir un contact électrique avec l'au moins un élément capteur (310) de l'agencement de capteur (120).

8. Dispositif (105) selon l'une quelconque des revendications précédentes, dans lequel l'agencement de capteur (120) comprend un agencement à ultrasons ou un agencement d'anémométrie permettant de détecter le flux sanguin.

9. Dispositif (105) selon la revendication 8, dans lequel l'agencement d'anémométrie comprend un élément chauffant et un capteur de température, ou dans lequel l'agencement d'anémométrie comprend une source lumineuse et une photodiode.

10. Système d'assistance cardiaque (100) comportant une unité de tête (135), une section d'entrée (140) adjacente à l'unité de tête (135) permettant d'introduire un flux sanguin, une section de guidage (112) adjacente à la section d'entrée (140) permettant de conduire le flux sanguin, une section de sortie (150) adjacente à la section de guidage (112) permettant d'évacuer le flux sanguin, un boîtier (110) adjacent à la section de sortie (150), un agencement d'entraînement (155) disposé dans le boîtier (110) et un câble d'alimentation (160) permettant d'établir un contact électrique avec le système d'assistance cardiaque (100), **caractérisé en ce que** le système d'assistance cardiaque (100) comporte un dispositif (105) selon l'une quelconque des revendications 1 à 9.

11. Système d'assistance cardiaque (100) selon la revendication 10, dans lequel l'au moins un élément capteur de l'agencement de capteur (120) du dispositif (105) est disposé dans l'unité de tête (135) du système d'assistance cardiaque (100) et l'au moins un autre élément de capteur (310) de l'agencement de capteur (120) est disposé dans la structure de support (115) du dispositif (105) ou l'élément de capteur et l'autre élément de capteur (310) sont disposés sur des entretoises (125) différentes.

12. Utilisation du dispositif (105) implanté selon l'une quelconque des revendications 1 à 9 pour la détermination d'un débit cardiaque.

13. Procédé (500) de détermination d'un débit cardiaque à l'aide du dispositif (105) implanté selon l'une quelconque des revendications 1 à 9, dans lequel le procédé (500) comprend les étapes suivantes :
la fourniture (501) d'un signal de commande, dans lequel le signal de commande est configuré pour commander l'agencement de capteur (120) afin d'émettre un signal d'excitation permettant de détecter le flux sanguin ;
la réception (503) d'un signal de capteur fourni par l'agencement de capteur (120) ; et
la détermination (505) du débit cardiaque à l'aide du signal de capteur.
